# NEUE EUROPÄISCHE PATENTSCHRIFT

(11) **EP 1 392 223 B2**
(45) Veröffentlichungstag und Bekanntmachung des Hinweises auf die Entscheidung über den Einspruch: **29.08.2012**
(45) Hinweis auf die Patenterteilung: 23.09.2009
(21) Anmeldenummer: 03730001.9
(22) Anmeldetag: 09.05.2003
(51) Int. Cl.: A61K 8/97, A61K 8/81, A61K 8/37, A61Q 1/04, A61Q 1/10, A61Q 17/04

(54) **LIPIDHALTIGE KOSMETISCHE ZUBEREITUNG**
LIPID-CONTAINING COSMETIC PREPARATION
PREPARATION COSMETIQUE CONTENANT DES LIPIDES

(30) Priorität: 10.05.2002 DE 20207328 U
(43) Veröffentlichungstag der Anmeldung: 03.03.2004
(73) Patentinhaber: Schwan-STABILO Cosmetics GmbH & Co., 90562 Heroldsberg (DE)
(72) Erfinder: EMIG, Susanne, 90542 Eckental (DE)
(74) Vertreter: Leissler-Gerstl, Gabriele
(86) Internationale Anmeldenummer: PCT/EP2003/004883
(87) Internationale Veröffentlichungsnummer: WO 2003/094869

(56) Entgegenhaltungen:
- WO-A-00/47169
- WO-A-00/64304
- US-A- 5 013 473
- US-A- 6 120 781

## Beschreibung

Die Erfindung betrifft eine lipidhältige Zubereitung, insbesondere in Form einer geschmeidigen Paste, welche sich für kosmetische Anwendungen, insbesondere im Bereich der dekorativen Kosmetik, zum Färben und Verschönen der Haut, der Lippen und der Augenlider, eignet. Beispielhaft genannt seien hier Lippen- und Wangenrouge, Make-up oder Lidschatten. Sie kann auch als Lipgloss, als Mittel zur Fixierung für die Lippen, als Pflegegrundlage zur Pflege der Haut oder als Sonnenschutzmittel verwendet werden. Diese lipidhaltige Zubereitung liegt insbesondere in wasserfreier Form vor.

Zubereitungen der genannten Art enthalten üblicherweise Lipide, wie z.B. Fette, Öle, öllösliche Pflanzenauszüge und mittel- bis langkettige Fettsäuren und Wachse.

Daneben kann eine feste Phase enthalten sein, welche aus feinteiligen Füllstoffen und Färbemitteln besteht. Im Falle von Sonnenschutzmitteln können besonders feinteilige Pigmente, sog. Nanopigmente mit einer durchschnittlichen Teilchengröße zwischen 5 und 25 nm, Anwendung finden, welche auf der Haut transparent wirken und sie nicht mehr einfärben. Beispielhaft genannt seien hier Siliciumdioxid, Titandioxid und Zinkoxid.

Nachteilig bei derartigen Zubereitungen ist, dass sie sich von der Haut oder den Lippen, auf die sie bestimmungsgemäß aufgebracht wurden, auf andere Oberflächen übertragen lassen, z.B. auf Tassen, Gläser, Textilien oder andere Hautbezirke. Dies kann Spuren in Form eines farbigen Abdrucks oder eines Fettfilms hinterlassen. Solche Produkte weisen also eine ungenügende Haftung auf dem Untergrund auf, was dazu führt, dass Lippen- und Wangenrouge, Make-up, Lidschatten und auch Sonnenschutzmittel regelmäßig neu aufgetragen werden müssen. Da ölige Bestandteile meist sehr gut auf der Haut spreiten, wandern die Pigmente vom ursprünglichen Auftragsort zusammen mit geringen Mengen aus der Ölphase in die Feinfältelung der Haut der unmittelbaren Umgebung, was sehr störend auf den optischen Gesamteindruck wirkt.

In der Vergangenheit versuchte man dem bei Lippenstiften und Lippenrouge durch den Einsatz sog. "bromo acids", Farbstoffen, die substantiv auf die Haut aufziehen, zu begegnen.

Da diese Farbstoffe aber aufgrund unterschiedlicher pH-Werte der Haut individuell verschiedene und nicht vorhersagbare Tönungen ergaben und zudem die Einfärbungen oft tagelang anhielten, wurde dieser Weg alsbald wieder verlassen.

Nachdem Siliconöle und Siliconharze Eingang in die Kosmetik gefunden hatten, wurde versucht die Haftung auf der Haut und damit die Haltbarkeit von dekorativen Zubereitungen hierdurch zu verbessern. So sind seit etwa 1977 Lidschatten- und Lippenstifte in Form von Minen bekannt, die in spitzbare Umhüllungen eingegossen wurden, welche in der Lipidphase unter anderem eine Mischung aus Phenyltrimethicone (ein nicht flüchtiges Siliconöl) und Cyclomethicone (ein flüchtiges Siliconöl) enthielten. Ihnen folgten dann gleichartige Zubereitungen nach, die Cyclomethicone als alleinige Siliconkomponente enthielten. Diese Stiftmassen ließen sich - obwohl sie eine scheinbar feste Struktur aufwiesen - weich und geschmeidig, ähnlich einer pastösen Masse, auf die Haut auftragen. Nach dem Abdampfen des flüchtigen Silicons verblieb ein weicher, elastischer Film auf der Haut, der sehr gut haftete und nur minimal in die Umgebung des ursprünglichen Auftragsortes auswanderte. Das Prinzip der Kombination zweier Siliconöle, übertragen auf pastöse Zubereitungen, findet sich beispielsweise auch wieder in EP 0 756 864.

Trotz der sehr positiven Effekte bezüglich Haftung und Haltbarkeit können solche Zubereitungen, die Siliconöle enthalten, bei empfindlichen Anwendern nachteilige Effekte bewirken, wenn sie in unmittelbarer Augennähe angewendet werden. Gelangen nämlich selbst minimale Mengen von Siliconölen in das Auge oder den Bindehautsack, so können sie zu einem öligen Schleier auf der Linse und zu unangenehmen Reizungen, einem sog. "wind bum effect" führen.

Aufgabe der Erfindung war es daher, eine lipidhaltige Zubereitung, insbesondere in Form einer geschmeidigen Paste bereit zu stellen, welche sich für kosmetische Anwendungen, insbesondere im Bereich der dekorativen Kosmetik, zum Färben und Verschönen der Haut, der Lippen und der Augenlider eignet, die sich leicht auftragen lässt, gut haftet und lange hält, und die vom ursprünglichen Auftragsort nicht oder nur minimal in die unmittelbare Umgebung einwandert. Diese Zubereitung soll im wesentlichen frei sein von Siliconölen oder Siliconderivaten, um deren Nachteile im unmittelbaren Augenbereich zu vermeiden. Die Zubereitung soll zudem bei unterschiedlichen Aufbewahrungstemperaturen, die sich auf den unterschiedlichen Transportwegen und beim Anwender selbst ergeben können, lagerstabil sein und insbesondere keine Synäreseeffekte nach längerer Lagerung zeigen. Ferner soll die Zubereitung eine Viskosität im Bereich von 1,5 bis 12 Pas aufweisen, die sich auch über eine längere Lagerzeit nicht wesentlich verändert. Unter Lagerzeit soll hier der Zeitraum zwischen der Abgabe an den Handel und dem Verbrauch beim Abnehmer verstanden werden. Diese Zubereitung soll sich also im Vergleich zu Produkten nach dem Stand der Technik weich und geschmeidig auftragen lassen, auf den Lippen nicht spannen und diese nicht austrocknen, auf der Haut gut und anhaltend haften, sich möglichst nicht auf Gegenstände und Textilien oder andere Hautbereiche übertragen und in unmittelbarer Augennähe nicht zu Reizungen führen.

Die Aufgabe wird gelöst mit einer Zubereitung gemäß Anspruch 1. Die Zubereitung besteht aus einer Lipidphase, welche dadurch gekennzeichnet ist, dass sie eine Mischung eines Esters, erhalten aus der Reaktion einer langkettigen Fettsäure mit einem langkettigen Fettalkohol in Kombination mit einem flüssigen Polybuten mit einem Molekulargewicht zwischen 1.500 und 4.500 Dalton, bevorzugt zwischen 2.200 und 3.200 Dalton und einer Viskosität zwischen 3.500 und 5.000 mPas bei 100 °C resp. größer als 18.000 mPas bei 40 °C, enthält.

Es wurde überraschenderweise gefunden, dass die Kombination aus einem langkettigen Ester und einem fließfähigem Polybuten eine Zusammensetzung liefert, die gut aufgetragen werden kann und am aufgetragenen Ort haftet, ohne in andere Hautbereich zu wandern oder auf damit in Berührung kommende Gegenstände übertragen zu werden. Die Kombination aus langkettigem Ester und fließfähigem Polybuten ist die wesentliche Komponente der erfindungsgemäßen Zubereitung. Die Zubereitung kann daneben übliche Inhaltsstoffe in an sich bekannter Menge enthalten, wobei jedoch die Verwendung von Wachsen, Lanolin und Lanolinderivaten ausgeschlossen ist.

Die Dokumente US-A-612 0781 und WO-A-0 047 169 beschreiben Lippenpflegezusammensetzungen, die Jojobaöl, Polybuten und Wachs enthalten.

Die eine wichtige Komponente der erfindungsgemäßen Zubereitung ist ein Ester, der durch Reaktion einer langkettigen Fettsäure mit einem langkettigen Fettalkohol erhalten wurde. Die langkettige Fettsäure ist dabei eine lineare, gesättigte oder einfach oder mehrfach ungesättigte Fettsäure mit einer Kettenlänge von 14 bis 36 Kohlenstoffatomen, bevorzugt 18 bis 26 Kohlenstoffatomen. Als langkettiger Fettalkohol wird ein linearer, gesättigter oder einfach oder mehrfach ungesättigter Alkohol mit einer Kettenlänge von 16 bis 36 Kohlenstoffatomen, bevorzugt 18 bis 26 Kohlenstoffatomen eingesetzt. Beispiele für erfindungsgemäß geeignete Ester sind z.B. die Ester der Palmitinsäure, Palmitoleinsäure, Stearinsäure, Ölsäure, Gadoleinsäure, Behensäure, Erucasäure, Eicosansäure, Eicosensäure, Linolsäure, Nervonsäure oder Melissinsäure mit Oleylalkohol, Behenylalkohol, Lignocerylalkohol, Cerylalkohol oder Myricylalkohol. Besonders bevorzugt findet Buxus Chinensis (Jojobaöl) Verwendung.

Der langkettige Ester wird einem Anteil von 10 bis 65 Gew.-%, bezogen auf das Gewicht der Zubereitung, bevorzugt 20 bis 45 Gew.-% eingesetzt.

Zur Verbesserung der inneren Struktur kann ein hydriertes Pflanzenöl, beispielsweise Hydrogenated Cottonseed Oil, Hydrogenated Castor Oil, Hydrogenated Vegetable Oil, Hydrogenated Rapeseed Oil oder Hydrogenated Coco-Glycerides zugesetzt werden und dies ist auch bevorzugt. Weiterhin können zur Erhöhung der Viskosität und zur Stabilisierung über einen breiteren Temperaturbereich wird Silica (amorphe Kieselsäure), Bentonite, Hectorite, Montmorillonite oder dgl. zugesetzt werden. Daneben können noch in der Kosmetik übliche Zusätze wie Konservierungsmittel, Antioxidantien, Riechstoffe, Vitamine, Sonnenschutzfilter und dgl. zugesetzt werden. Diese Mittel werden in den üblicherweise bekannten Mengen verwendet. Beispiele für Konservierungsmittel sind Parabene, wie Methylparaben und Propylparaben. Beispiele für Antioxidantien bzw. Vitamine sind Tocopherol und Ascorbyl Palmitat.

Der andere erfindungsgemäß wesentliche Bestandteil ist ein fließfähiges Polybuten. Als Polybuten können die an sich bekannten Polymere eingesetzt werden, die ein Molekulargewicht in einem solchen Bereich aufweisen, dass sie fließfähig sind. Dies ist üblicherweise bei einem Molekulargewicht von 1500 bis 4000 Dalton der Fall. Bevorzugt werden Polybutene mit einem Molekulargewicht von 2200 bis 3200 Dalton eingesetzt. Damit die Zubereitung die zum Auftragen geeignete Viskosität hat, sollte der Wert der Viskosität des Polybutens zwischen 3500 und 5000 mPas bei 100 °C bzw. mehr als 18.000 mPas bei 40 °C liegen. Die Viskosität wird in an sich bekannter Weise bestimmt, z.B. mit einem Meßsystem Platte/Platte mit einem Plattendurchmesser von 2 cm und einem Plattenabstand von 400 µm bei einem Schergeschwindigkeitsgefälle von 628 s⁻¹.

Der Gehalt an langkettigen Estern und Polybuten liegt bei jeweils 10 bis 65 Gew.-%, bevorzugt bei 20 bis 45 Gew.-%, sofern es sich um färbemittelhaltige Formulierungen handelt, wobei das Mengenverhältnis untereinander bei 2 : 1 bis 1 : 2 liegt. Der Gehalt an hydriertem Pflanzenöl, falls vorhanden, liegt in einem Bereich von 0,5 bis 10 Gew.-%, bevorzugt zwischen 1,5 und 5,5 Gew.-%. Der Gehalt an Stabilisierungsmittel, wie z.B. Silica, Bentonite, Hectorite, Montmorillonite und dgl. liegt, falls vorhanden, allgemein in einem Bereich zwischen 0,3 und 8 Gew.-%, bevorzugt in einem Bereich zwischen 1 und 5 Gew.-%.

Die vorgenannte feste Phase kann aus Füllstoffen, wie z.B. Talkum, Kaolin, Stärke und modifizierte Stärke, Polytetrafluorethylenpulver (Teflon), Nylonpulver, Bornitrid, aus unlöslichen Metallseifen, wie Mg-Stearat, Ca-Stearat, Sr-Stearat, Zn-Stearat und aus anorganischen oder organischen Pigmenten bestehen. Für letztere seien beispielhaft genannt: Titandioxid, Zinkoxid, Eisenoxide, Chromoxid, Chromoxidhydrat, Ultramarin, Berliner Blau (Ferric Blue), Glimmer, Perlglanzmittel wie z.B. mit Titandioxid beschichtete Glimmer, farbige, mit Titandioxid und Metalloxiden beschichtete Glimmer, Bismuthoxidchlorid, beschichtetes Bismuthoxidchlorid, plättchenförmige Metallpulver von Aluminium, Messing, Bronze, Kupfer, Silber, Gold, sowie Verlackungen organischer Färbemittel mit Aluminium, Barium, Calcium oder Strontium. Diese Aufzählung ist nur beispielhaft und nicht abschließend. Diese Zusätze erfolgen mit der Maßgabe, dass sie von der jeweiligen nationalen oder regionalen Kosmetik-Gesetzgebung auch zugelassen sind. Auch die Einsatzmengen liegen im Rahmen der durch die jeweilige Kosmetik-Gesetzgebung erlaubten Höchstmenge. Die Mengenanteile an Pigmenten liegen dabei in einem Bereich von 0 bis 25 Gew.-%, vorzugsweise in einem Bereich von 5 bis 20 Gew.-% und ganz besonders bevorzugt in einem Bereich von 8 bis 15 Gew.-%.

Gegenstand der Erfindung ist ferner eine lipidhaltige Zubereitung, welche frei ist von flüssigen Triglyceriden. Verwendung finden bevorzugt ausschließlich feste, hydrierte Triglyceride mit einem Schmelzbereich zwischen 30 und 90 °C. Beispielhaft genannt seien hier Hydrogenated Cotton Seed Oil, Hydrogenated Coco-Glycerides, Hydrogenated Castor Oil, Hydrogenated Rapessed Oil und Hydrogenated Vegetable Oil. Die Bezeichnung der Rohstoffe erfolgt mit den dem einschlägig befaßten Fachmann geläufigen "INCI-Namen". Falls hydrierte Triglyzeride, insbesondere hydrierte Pflanzenöle verwendet werden, werden diese bevorzugt in einem Anteil von 0,5 bis 10 Gew.-%, besonders bevorzugt 1,5 bis 5,5 Gew.-%, bezogen auf das Gewicht der Zubereitung zugegeben.

Auf den Einsatz von natürlichen oder synthetischen Wachsen wird in den erfindungsgemäßen Zubereitungen verzichtet, wie auf die Verwendung von Lanolin oder Lanolinderivaten. Dabei werden unter Wachsen pflanzliche, tierische und/oder synthetische Wachse verstanden. Wachse können in der Zubereitung während der Lagerzeit Kristallite und minimale Kristallstrukturen bilden, welche dann die Viskosität - zwar reversibel aber für den Verbraucher merkbar - verändern können. Lanolin und Lanolinderivate können, wegen ihrer wenigstens teilweise ausgeprägten selbstemulgierenden Eigenschaften, zusammen mit der Feuchtigkeit der Haut spontane W/O-Emulsionen bilden, welche die Haftung und Haltbarkeit auf der Haut negativ beeinflussen können.

In einer bevorzugten Ausführungsform ist die lipidhaltige Zubereitung wasserfrei und liegt in Form einer geschmeidigen Paste vor. Diese wasserfreie, geschmeidige Paste hat bevorzug eine dynamische Viskosität im Bereich von 1,5 bis 12 mPa·s, wobei die dynamische Viskosität wie oben beschrieben bestimmt werden kann.

Die Erfindung betrifft geschmeidige lipidhaltige Zubereitungen, die auf die Haut, die Semischleimhäute oder in der Nähe von Schleimhäuten, beispielsweise in der Nähe der Augen, aufgetragen werden. Als Semischleimhäute sollen hier insbesondere die Lippen verstanden werden. Beispielhaft genannt seien Zubereitungen zum Färben oder zur Pflege der Lippen, zur Erhöhung des Glanzes, insbesondere Lipgloss, zur Fixierung von Lippenstift, Zubereitungen zum Färben oder zur Pflege der Haut, wie beispielsweise Make-up, Rouge, Camouflage zum Kaschieren von Altersflecken oder Rosacea, Concealer und dgl., ferner Sonnenschutzprodukte mit unterschiedlichen Lichtschutzfaktoren (SPF), bis hin zu sog. Sunblockern unter Verwendung von sehr feinteiligen Nanopigmenten oder bunt eingefärbten, höher pigmentierten Sunblockem, welche als Körperbemalung bei Surfern und Windsurfem beliebt sind.

Die Zubereitung kann auch ohne Zusatz von Färbemitteln hergestellt werden und ggf. sog. kosmetische Wirkstoffe enthalten. Sie findet dann Verwendung als Lipgloss oder als Fixierungsmittel, welche über einen Lippenstift aufgetragen werden. Enthält diese uneingefärbte Zubereitung Lichtschutzfilter, so kann sie als Lippenschutz und -pflege Verwendung finden. Die Haut der Lippen enthält ja bekanntermaßen, im Gegensatz zur Haut des Körpers, keine Pigmentierung. Geeignete öllösliche Lichtfiltersubstanzen, die im Bereich des UV-A und UV-B guten Schutz bieten, sind dem einschlägig befassten Fachmann in ausreichender Zahl bekannt und durch die jeweilige nationale oder regionale Gesetzgebung z.B. in der EU, in Japan und in den U.S.A. geregelt - in Deutschland beispielsweise durch die Anlage 7 zu § 3b der Kosmetik-Verordnung und sollen hier deshalb nicht umfassend aufgelistet werden. Beispielhaft erwähnt seien deshalb nur das Isoamyl p-Methoxycinnamate als UV-B Filter und 4-Methylbenzylidene Camphor als UV-A Filter.

Die erfindungsgemäße Zubereitung liegt vor in Form einer weichen geschmeidigen Paste, welche sich leicht und gleichmäßig applizieren und verteilen lässt. Sie bildet eine angenehm empfundene, gut haftende Schicht auf den vorgenannten Hautbereichen, welche nicht trocknet und den ganzen Tag dort verbleiben kann. Sie kann in den Anwendern bekannter Weise wieder von der Haut entfernt werden - durch geeignete Abschminkmittel oder -tücher oder durch Waschen mit Feinseife oder geeigneten milden Tensidzubereitungen. Sie kann in bekannter Weise in geeignete Behältnisse, wie Flaschen, ggf. mit Spatel, Tiegel oder Tuben abgefüllt und daraus vom Anwender wieder entnommen werden. Sie kann aber auch, wegen der damit verbundenen verbesserten hygienischen Verhältnisse, in geeignete Auftragvorrichtungen, sog. Spendermechaniken eingebracht und daraus appliziert werden. Für den Auftrag kleiner Mengen, wie sie z.B. für die Applikation im Lippen- oder Augenbereich benötigt werden, bieten sich Auftragvorrichtungen an, wie sie z.B. aus US 6,238,117 oder aus US 6,309,128 bekannt sind, da diese eine sehr schöne Feindosierung erlauben.

Die erfindungsgemäße Zubereitung hat eine solche Konsistenz, dass sie sehr leicht in wiederverschließbaren Flaschen, Tiegeln oder Tuben abgefüllt werden kann. Besonders geeignet ist sie zur Verwendung in einer sogenannten Spendermechanik. Gegenstand der Erfindung ist daher auch die Verwendung der erfindungsgemäßen lipidhaltigen Zubereitung zur Befüllung von wiederverschließbaren Flaschen, Tiegeln oder Tuben und insbesondere als Füllung einer sogenannten Spendermechanik.

Die erfindungsgemäße Zubereitung soll anhand der nachfolgenden Beispiele im Detail erläutert werden, welche sie jedoch nicht abschließend beschreiben. Dabei erfolgen alle Mengenangabe in Gewichtsprozent (Gew.-%), bezogen auf das Gesamtgewicht der Zubereitung:

### Beispiel 1 - pastenförmiges Lippenrouge

| | |
|---|---|
| Jojoba-Öl | 39,500 |
| Polybutene | 33,300 |
| Pigments | 18,000 |
| Hydrogenated Cottonseed Oil | 4,000 |
| Silica | 3,500 |
| Tocopherol | 0,850 |
| Fragrance | 0,300 |
| Methylparaben | 0,200 |
| Propylparaben | 0,150 |
| Ascorbyl Palmitate | 0,200 |

Die Herstellung erfolgt, indem man Buxus Chinensis, Polybutene und Hydrogenated Cottonseed Oil in einer geeigneten Homogenisiermaschine mit Ankerrührwerk und Zahnkranzhomogenisator vorlegt und auf etwa 80 °C erwärmt. Anschließend wird das Silica eingestreut und mittels Homogenisator dispergiert. Anschließend werden die Pigmente zugesetzt, die Mischung wird dann unter hohem Scherkrafteintrag homogenisiert, um alle Pigmentagglomerate zu zerstören. Die Masse wird dann durch Anlegen von Vakuum entlüftet. Anschließend werden die Konservierungsmittel und Antioxidantien zu der noch heißen Mischung zugesetzt, und es wird kurz nachhomogenisiert. Unter gutem Rühren mit dem Ankerrührwerk wird die Mischung dann abgekühlt auf etwa 45-50 °C. Bei dieser Temperatur werden dann das Tocopherol und die Riechstoffmischung (Fragrance) zugesetzt. Anschließend wird unter Rühren mit dem Ankerrührwerk weiter gekühlt bis etwa 35 °C. Die jetzt pastöse Mischung wird nun in den Abfüllkessel überführt und ohne weitere Maßnahmen auf Raumtemperatur abkühlen lassen. Anschließend wird auf einer Abfüll- oder Montagemaschine in die geeigneten Behältnisse abgefüllt, nachdem die Zubereitung von der Qualitätssicherung überprüft und freigegeben wurde. Man erhält eine geschmeidige, weiche Paste mit einer Viskosität von 4.500 mPas.

### Beispiel 2 - Cremelidschatten

| | |
|---|---|
| Jojoba-Öl | 45,000 |
| Polybutene | 27,750. |
| Pigments | 9,000 |
| Mica (and) Titanium Dioxide | 10,000 |
| Hydrogenated Cottonseed Oil | 4,000 |
| Silica | 3,000 |
| Tocopherol | 0,500 |
| Fragrance | 0,200 |
| Methylparaben | 0,200 |
| Propylparaben | 0,150 |
| Ascorbyl Palmitate | 0,200 |

Die Herstellung erfolgt analog zu Beispiel 1, jedoch werden hierbei zunächst die Färbemittel bei etwa 80 °C zu der Fettphase zugegeben und homogen eingearbeitet, danach werden die Perlglanzmittel zugesetzt und der dann nochmals kurz homogenisiert. Danach wird unter gutem Rühren abgekühlt und bei 45 bis 50 °C die Riechstoffmischung und das Tocopherol zugesetzt. Anschließend wird weiterverfahren, wie oben beschrieben. Man erhält eine weiche, geschmeidige Paste mit schönem Perlglanz und einer Viskosität von 3,600 mPas.

### Beispiel 3 - Lipgloss

| | |
|---|---|
| Jojoba-Öl | 41,000 |
| Polybutene | 47,800 |
| Hydrogenated Cottonseed Oil | 4,000 |
| Silica | 2,500 |
| Isoamyl p-Methoxycinnamate | 2,000 |
| 4-Methylbenzylidene Camphor | 1,000 |
| Tocopherol | 0,900 |
| Fragrance | 0,250 |
| Methylparaben | 0,200 |
| Propylparaben | 0,150 |
| Ascorbyl Palmitate | 0,200 |

Die Herstellung erfolgt im wesentlichen analog zu den vorstehend beschriebenen Verfahren, wobei die Lichtfiltersubstanzen zusammen mit Tocopherol und Riechstoffmischung bei etwa 45-50 °C zugesetzt werden. Man erhält eine ungefärbte, transparente, sehr weiche Paste, mit einer Viskosität von 2.400 mPas.

### Beispiel 4 - Sunblocker für Surfer

| | |
|---|---|
| Jojoba-Öl | 40,000 |
| Polybutene | 29,500 |
| Titanium Dioxide (Nanopigment) | 10,000 |
| iron Oxides (Rot und Gelb) | 5,000 |
| Hydrogenated Cottonseed Oil | 4,000 |
| Silica | 3,500 |
| Isoamyl p-Methoxycinnamate | 3,500 |
| 4-Methylbenzylidene Camphor | 2,500 |
| Tocopherol | 1,200 |
| Fragrance | 0,250 |
| Methylparaben | 0,200 |
| Propylparaben | 0,150 |
| Ascorbyl Palmitate | 0,200 |

Die Herstellung erfolgt nach dem unter Beispiel 1 angegebenen Verfahren. Man erhält eine rotorange, weiche Paste mit einer Viskosität von 3.800 mPas. Der Lichtschutzfaktor (SPF) dieser Zubereitung liegt oberhalb von 25.

## Patentansprüche

1. Lipidhaltige Zubereitung, insbesondere für kosmetische Anwendungen, **dadurch gekennzeichnet, dass** sie eine Mischung aus einem Ester und einem flüssigen Polybuten enthält, wobei der Ester das Reaktionsprodukt einer linearen langkettigen gesättigten oder einfach oder mehrfach ungesättigten Fettsäure mit einer Kettenlänge zwischen C-14 und C-36 mit einem linearen langkettigen gesättigten oder einfach oder mehrfach ungesättigten Fettalkohol oder Wachsalkohol mit einer Kettenlänge zwischen C-16 und C-36 ist, und wobei das Polybuten ein Molekulargewicht zwischen 1.500 und 4.500 Dalton und eine Viskosität zwischen 3.500 und 5.000 mPas bei 100° C bzw. größer als 18.000 mPas bei 40° C aufweist, wobei die Zubereitung frei ist von pflanzlichen, tierischen und/oder synthetischen Wachsen und/oder deren Ersatzstoffen, die einen Tropfpunkt zwischen 40° C und 130° C haben und von Lanolin und seinen Derivaten.

2. Lipidhaltige Zubereitung nach Anspruch 1, **dadurch gekennzeichnet, dass** es sich bei dem linearen langkettigen Ester um Jojobaöl handelt.

3. Lipidhaltige Zubereitung nach einem der vorhergehenden Ansprüche, **dadurch gekennzeichnet, dass** sie zusätzlich ein hydriertes Pflanzenöl enthält.

4. Lipidhaltige Zubereitung nach einem der vorhergehenden Ansprüche, **dadurch gekennzeichnet, dass** sie zusätzlich ein Stabilisierungsmittel, Färbemittel und/oder mindestens ein Lichtschutzmittel enthält.

5. Lipidhaltige Zubereitung nach einem der vorhergehenden Ansprüche, **dadurch gekennzeichnet, dass** sie zusätzlich weitere Zusätze, wie Füllstoffe, Konservierungsmittel, Antioxidantien und Riechstoffe enthält.

6. Verwendung einer lipidhaltigen Zubereitung nach einem der vorhergehenden Ansprüche zur Verwendung im Bereich der dekorativen Kosmetik zum Pflegen, Färben und Verschönen der Haut, der Lippen und der Augenlider.

7. Verwendung einer lipidhaltigen Zubereitung nach Anspruch 6, **dadurch gekennzeichnet, dass** es sich um ein Rouge, Lippenrouge, Make-up, Camouflage oder einen Concealer handelt.

8. Verwendung einer lipidhaltigen Zubereitung nach Anspruch 6, **dadurch gekennzeichnet, dass** sie als Lippenpflege, als Mittel zur Fixierung für die Lippen, als Pflegegrundlage, als Mittel zur Pflege der Haut oder als Sonnenschutzmittel vorliegt.

9. Lipidhaltige Zubereitung nach einem der vorhergehenden Ansprüche, **dadurch gekennzeichnet, dass** sie in wasserfreier Form vorliegt.

10. Lipidhaltige Zubereitung nach Anspruch 9, **dadurch gekennzeichnet, dass** sie in Form einer wasserfreien, geschmeidigen Paste vorliegt.

11. Lipidhaltige Zubereitung nach Anspruch 10, **dadurch gekennzeichnet, dass** die wasserfreie, geschmeidige Paste eine dynamische Viskosität in einem Bereich von 1,5 bis 12 Pas aufweist.

12. Lipidhaltige Zubereitung nach Anspruch 11, **dadurch gekennzeichnet, dass** die dynamische Viskosität der wasserfreien, geschmeidigen Paste mit einem Meßsystem Platte/Platte mit Plattendurchmesser 2 cm und Plattenabstand 400 µm bei einem Schergeschwindigkeitsgefälle von 628 1/s gemessen wurde.

13. Lipidhaltige Zubereitung nach einem der vorausgehenden Ansprüche, **dadurch gekennzeichnet, dass** der Gehalt an linearem, langkettigen Ester und flüssigem Polybuten bei jeweils 10 Gew.-% bis 65 Gew.-%, bevorzugt bei 20 bis 45 Gew.-% in färbemittelhaltigen Zubereitungen, liegt und wobei das Mengenverhältnis untereinander bei 2 : 1 bis 1 : 2 liegt.

14. Lipidhaltige Zubereitung nach einem der vorausgehenden Ansprüche, **dadurch gekennzeichnet, dass** sie feste hydrierte Triglyceride mit einem Schmelzbereich von 30 °C bis 90 °C enthält.

15. Lipidhaltige Zubereitung nach einem der vorausgehenden Ansprüche, **dadurch gekennzeichnet, dass** es sich bei den festen hydrierten Triglyceriden um hydrierte Pflanzenöle mit einem Schmelzbereich von 30 °C bis 90 °C handelt.

16. Lipidhaltige Zubereitung nach einem der vorausgehenden Ansprüche, **dadurch gekennzeichnet, dass** der Gehalt an hydriertem Pflanzenöl in einem Bereich von 0,5 bis 10 Gew.-%, bevorzugt in einem Bereich von 1,5 bis 5,5 Gew.-% liegt.

17. Lipidhaltige Zubereitung nach einem der vorausgehenden Ansprüche, **dadurch gekennzeichnet, dass** als Stabilisierungsmittel Silica, Bentonite, Hectorite, Montmorillonite oder Gemische derselben enthalten sind.

18. Lipidhaltige Zubereitung nach Anspruch 17, **dadurch gekennzeichnet, dass** der Gehalt an Silica, Bentonite, Hectorite, Montmorillonite oder deren Gemischen bei 0,3 bis 8 Gew.-%, bevorzugt bei 1 bis 5 Gew.-% liegt.

19. Lipidhaltige Zubereitung nach einem der vorausgehenden Ansprüche, **dadurch gekennzeichnet, dass** sie zusätzlich eine Feststoffphase enthält, welche Füllstoffe, anorganische Pigmente, organische Pigmente oder deren Mischungen enthält.

20. Lipidhaltige Zubereitung nach Anspruch 19, **dadurch gekennzeichnet, dass** es sich bei den Füllstoffen um Talkum, Kaolin, Stärke, modifizierte Stärke, Polytetrafluorethylenpulver, Nylonpulver, Bornitrid, Mg-Stearat, Ca-Stearat, Sr-Stearat, Zn-Stearat oder deren Mischungen handelt.

21. Lipidhaltige Zubereitung nach Anspruch 19, **dadurch gekennzeichnet, dass** es sich bei den anorganischen Pigmenten um Titandioxid, Zinkoxid, Eisenoxide, Chromoxid, Chromoxidhydrat, Ultramarin, Berliner Blau (Ferric Blue), Glimmer, mit Titandioxid beschichtete Glimmer, mit Titandioxid und mit Metalloxiden beschichtete Glimmer, Bismuthoxidchlorid, beschichtetes Bismuthoxidchlorid, plättchenförmige Metallpulver von Aluminium, Messing, Bronze, Kupfer, Silber, Gold oder deren Mischungen handelt.

22. Lipidhaltige Zubereitung nach Anspruch 19, **dadurch gekennzeichnet, dass** es sich bei den organischen Pigmenten um Verlackungen organischer Färbemittel mit Aluminium, Barium, Calcium, Strontium, Zirkon und um deren Mischungen handelt.

23. Lipidhaltige Zubereitung nach Anspruch 21 oder 22, **dadurch gekennzeichnet, dass** die Mengenanteile an Pigmenten in einem Bereich von 0,1 bis 25 Gew.-%, bevorzugt in einem Mengenbereich von 5 bis 20 Gew.-%, ganz besonders bevorzugt in einem Bereich von 8 bis 15 Gew.-% liegen.

24. Lipidhaltige Zubereitung nach einem der vorausgehenden Ansprüche, **dadurch gekennzeichnet, dass** sie bei Verwendung als Lichtschutzmittel Titandioxid und/oder Zinkoxid als sog. Nanopigmente mit Teilchengrößen im Bereich von 5 bis 25 nm enthält.

25. Lipidhaltige Zubereitung nach Anspruch 24, **dadurch gekennzeichnet, dass** sie die sog. Nanopigmente in Kombination mit üblichen, durch die jeweilige nationale oder regionale Gesetzgebung zugelassene, öllösliche UV-A- und UV-B-Lichtfiltersubstanzen enthält.

26. Lipidhaltige Zubereitung nach Anspruch 25, **dadurch gekennzeichnet, dass** sie in Kombination mit sog. Nanopigmenten als UV-A- und UV-B-Lichtfiltersubstanzen bevorzugt 4-Methylbenzylidene Camphor und Isoamyl p-Methoxycinnamate enthält.

27. Produkt, das eine lipidhaltige Zubereitung nach einem der vorausgehenden Ansprüche in wiederverschließbare Flaschen, Tiegel oder Tuben abgefüllt, aufweist.

28. Produkt, das eine lipidhaltige Zubereitung nach einem der Ansprüche 1 bis 21 in eine wiederverschließbare, sog. Spendermechanik abgefüllt, aufweist.

## Claims

1. A lipid-containing preparation, in particular for cosmetic uses, **characterised in that** it contains a mixture of an ester and a liquid polybutene, wherein the ester is the reaction product of a linear long-chain saturated or singly or multiply unsaturated fatty acid with a chain length of between C-14 and C-36 with a linear long-chain saturated or singly or multiply unsaturated fatty alcohol or wax alcohol with a chain length of between C-16 and C-36, and wherein the polybutene has a molecular weight of between 1500 and 4500 daltons and a viscosity of between 3500 and 5000 mPas at 100°C or greater than 18000 mPas at 40°C, wherein the preparation is free of vegetable, animal and/or synthetic waxes and/or their substitutes having a dropping point of between 40°C and 130°C and/or lanolin and derivatives thereof.

2. A lipid-containing preparation according to claim 1, **characterised in that** the linear long-chain ester is jojoba oil.

3. A lipid-containing preparation according to one of the preceding claims, **characterised in that** it additionally contains a hydrogenated vegetable oil.

4. A lipid-containing preparation according to one of the preceding claims, **characterised in that** it additionally contains a stabilisation agent, colouring agent and/or at least one light-protection agent.

5. A lipid-containing preparation according to one of the preceding claims, **characterised in that** it additionally contains further additives such as fillers, preserving agents, antioxidants and fragrances.

6. Use of a lipid-containing preparation according to one of the preceding claims for use in the area of decorative cosmetics for care of, colouring and improving the appearance of the skin, the lips and the eyelids.

7. Use of a lipid-containing preparation according to claim 6, **characterised in that** it is a rouge, lip rouge, make-up, camouflage or a concealer.

8. Use of a lipid-containing preparation according to claim 6, **characterised in that** it is in the form of lip care, a fixing agent for the lips, a care foundation, an agent for caring for the skin or a sun-protection agent.

9. A lipid-containing preparation according to one of the preceding claims, **characterised in that** it is in water-free form.

10. A lipid-containing preparation according to claim 9, **characterised in that** it is in the form of a water-free workable paste.

11. A lipid-containing preparation according to claim 10, **characterised in that** the water-free workable paste has a dynamic viscosity in a range of 1.5 to 12 Pas.

12. A lipid-containing preparation according to claim 11, **characterised in that** the dynamic viscosity of the water-free workable paste was measured with a plate/plate measuring system with a plate diameter of 2 cm and a plate spacing of 400 µm at a shearing speed gradient of 628 1/s.

13. A lipid-containing preparation according to one of the preceding claims, **characterised in that** the content of linear long-chain ester and liquid polybutene is respectively 10% by weight to 65% by weight, preferably 20 to 45% by weight in colouring agent-containing preparations, and wherein the quantitative ratio with each other is 2:1 to 1:2.

14. A lipid-containing preparation according to one of the preceding claims, **characterised in that** it contains solid hydrogenated triglycerides with a melting range of 30°C to 90°C.

15. A lipid-containing preparation according to one of the preceding claims, **characterised in that** the solid hydrogenated triglycerides are hydrogenated vegetable oils with a melting range of 30°C to 90°C.

16. A lipid-containing preparation according to one of the preceding claims, **characterised in that** the content of hydrogenated vegetable oil is in a range of 0.5 to 10% by weight, preferably in a range of 1.5 to 5.5% by weight.

17. A lipid-containing preparation according to one of the preceding claims, **characterised in that** silica, bentonite, hectorite, montmorillonite or mixtures thereof are contained therein as the stabilising agent.

18. A lipid-containing preparation according to claim 17, **characterised in that** the content of silica, bentonite, hectorite, montmorillonite or mixtures thereof is 0.3 to 8% by weight, preferably 1 to 5% by weight.

19. A lipid-containing preparation according to one of the preceding claims, **characterised in that** it additionally contains a solid phase which contains fillers, inorganic pigments, organic pigments or mixtures thereof.

20. A lipid-containing preparation according to claim 19, **characterised in that** the fillers are talcum, kaolin, starch, modified starch, polytetrafluoroethylene powder, nylon powder, boron nitride, Mg stearate, Ca stearate, Sr stearate, Zn stearate or mixtures thereof.

21. A lipid-containing preparation according to claim 19, **characterised in that** the inorganic pigments are titanium dioxide, zinc oxide, iron oxides, chromium oxide, chromium hydroxide, ultramarine, Berlin Blue (ferric blue), mica, mica coated with titanium dioxide, mica coated with titanium dioxide and with metal oxides, bismuth oxide chloride, coated bismuth oxide chloride, metal powders in flake form of aluminium, brass, bronze, copper, silver, gold or mixtures thereof.

22. A lipid-containing preparation according to claim 19, **characterised in that** the organic pigments involve lakings of organic colouring agents with aluminium, barium, calcium, strontium, zirconium and mixtures thereof.

23. A lipid-containing preparation according to claim 21 or claim 22, **characterised in that** the quantitative proportions of pigments are in a range of 0.1 to 25% by weight, preferably in a quantitative range of 5 to 20% by weight, quite particularly preferably in a range of 8 to 15% by weight.

24. A lipid-containing preparation according to one of the preceding claims, **characterised in that** when used as a light-protection agent it contains titanium dioxide and/or zinc oxide in the form of so-called nanopigments with particle sizes in the range of 5 to 25 nm.

25. A lipid-containing preparation according to claim 24, **characterised in that** it contains the so-called nanopigments in combination with usual, oil-soluble UV-A- and UV-B-light-filter substances approved by the respective national or regional legislation.

26. A lipid-containing preparation according to claim 25, **characterised in that** it contains 4-methylbenzylidene camphor and isoamyl p-methoxycinnamates in combination with so-called nanopigments as UV-A- and UV-B-light-filter substances.

27. A product which has a lipid-containing preparation according to one of the preceding claims filled into reclosable bottles, pots or tubes.

28. A product which has a lipid-containing preparation according to one of claims 1 to 21 filled into a reclosable, so-called dispenser mechanism.

## Revendications

1. Préparation contenant des lipides, en particulier pour des applications cosmétiques, **caractérisée en ce qu'**elle contient un mélange d'un ester et d'un polybutène liquide, où l'ester est le produit de la réaction d'un acide gras linéaire à chaîne longue saturé ou mono- ou poly-insaturé ayant une longueur de chaîne comprise entre C-14 et C-36 avec un alcool gras ou un alcool de cire linéaire à chaîne longue saturé ou mono- ou poly-insaturé ayant une longueur de chaîne comprise entre C-16 et C-36, et où le polybutène présente un poids moléculaire compris entre 1 500 et 4 500 daltons et une viscosité comprise entre 3 500 et 5 000 mPa.s à 100 °C ou supérieure à 18 000 mPa.s à 40 °C, où la préparation est exempte de cires végétales, animales et/ou synthétiques et/ou de leurs succédanés ayant un point de goutte compris entre 40 °C et 130 °C et/ou de lanoline et de ses dérivés.

2. Préparation contenant des lipides selon la revendication 1, **caractérisée en ce qu'**il s'agit, concernant l'ester linéaire à chaîne longue, d'huile de jojoba.

3. Préparation contenant des lipides selon l'une des revendications précédentes, **caractérisée en ce qu'**elle contient en outre une huile végétale hydrogénée.

4. Préparation contenant des lipides selon l'une des revendications précédentes, **caractérisée en ce qu'**elle contient en outre un agent stabilisant, des colorants et/ou au moins un agent de protection contre la lumière.

5. Préparation contenant des lipides selon l'une des revendications précédentes, **caractérisée en ce qu'**elle contient en outre d'autres additifs, comme des matières de charge, des conservateurs, des antioxydants et des substances odoriférantes.

6. Utilisation d'une préparation contenant des lipides selon l'une des revendications précédentes pour une utilisation dans le domaine de la cosmétique de maquillage pour le soin, la coloration et la protection de la peau, des lèvres et des paupières.

7. Utilisation d'une préparation contenant des lipides selon la revendication 6, **caractérisée en ce qu'**il s'agit d'un rouge, d'un rouge à lèvres, d'un maquillage, d'un maquillage correcteur ou d'un concealer ou un agent couvrant.

8. Utilisation d'une préparation contenant des lipides selon la revendication 6, **caractérisée en ce qu'**elle se présente sous la forme d'un soin des lèvres, d'un agent fixateur pour les lèvres, d'une base de soin, d'un agent de soin de la peau ou d'un agent de protection solaire.

9. Préparation contenant des lipides selon l'une des revendications précédentes, **caractérisée en ce qu'**elle se présente sous une forme anhydre.

10. Préparation contenant des lipides selon la revendication 9, **caractérisée en ce qu'**elle se présente sous la forme d'une pâte anhydre onctueuse.

11. Préparation contenant des lipides selon la revendication 10, **caractérisée en ce que** la pâte anhydre onctueuse présente une viscosité dynamique dans une plage de 1,5 à 12 Pa.s.

12. Préparation contenant des lipides selon la revendication 11, **caractérisée en ce que** la viscosité dynamique de la pâte anhydre onctueuse a été mesurée avec un système de mesure plaque/plaque présentant un diamètre de plaque de 2 cm et un écart entre plaques de 400 µm, avec un gradient de cisaillement de 628 1/s.

13. Préparation contenant des lipides selon l'une des revendications précédentes, **caractérisée en ce que** la teneur en ester linéaire à chaîne longue et en polybutène liquide est respectivement de 10 % en poids à 65 % en poids, de préférence de 20 à 45 % en poids dans des préparations contenant des colorants, et où le rapport de quantités entre les deux est compris entre 2:1 et 1:2.

14. Préparation contenant des lipides selon l'une des revendications précédentes, **caractérisée en ce qu'**elle contient des triglycérides solides hydrogénés présentant une plage de fusion comprise entre 30 °C et 90 °C.

15. Préparation contenant des lipides selon l'une des revendications précédentes, **caractérisée en ce qu'**il s'agit, concernant les triglycérides solides hydrogénés, d'huiles végétales hydrogénées présentant une plage de fusion comprise entre 30 °C et 90 °C.

16. Préparation contenant des lipides selon l'une des revendications précédentes, **caractérisée en ce que** la teneur en huile végétale hydrogénée est comprise dans une plage de 0,5 à 10 % en poids, de préférence dans une plage comprise entre 1,5 et 5,5 % en poids.

17. Préparation contenant des lipides selon l'une des revendications précédentes, **caractérisée en ce que** sont contenus, en tant qu'agents stabilisants, de la silice, des bentonites, des hectorites, des montmorillonites ou des mélanges de celles-ci.

18. Préparation contenant des lipides selon la revendication 17, **caractérisée en ce que** la teneur en silice, bentonites, hectorites, montmorillonites ou en leurs mélanges est comprise entre 0,3 et 8 % en poids, de préférence entre 1 et 5 % en poids.

19. Préparation contenant des lipides selon l'une des revendications précédentes, **caractérisée en ce qu'**elle contient en outre une phase solide qui contient des matières de charge, des pigments inorganiques, des pigments organiques ou leurs mélanges.

20. Préparation contenant des lipides selon la revendication 19, **caractérisée en ce qu'**il s'agit, concernant les matières de charge, de talc, de kaolin, d'amidon, d'amidon modifié, de poudre de polytétrafluoroéthylène, de poudre de nylon, de nitrure de bore, de stéarate de magnésium, de stéarate de calcium, de stéarate de strontium, de stéarate de zinc, ou de leurs mélanges.

21. Préparation contenant des lipides selon la revendication 19, **caractérisée en ce qu'**il s'agit, concernant les pigments inorganiques, de dioxyde de titane, d'oxyde de zinc, d'oxydes de fer, d'oxyde de chrome, d'oxyde de chrome hydraté, de bleu outremer, de bleu de Prusse (bleu ferrique), de mica, de mica revêtu de dioxyde de titane, de mica revêtu de dioxyde de titane et d'oxydes métalliques, d'oxychlorure de bismuth, d'oxychlorure de bismuth revêtu, de poudre métallique en paillettes d'aluminium, de laiton, de bronze, de cuivre, d'argent, d'or ou de leurs mélanges.

22. Préparation contenant des lipides selon la revendication 19, **caractérisée en ce qu'**il s'agit concernant les pigments organiques de formations de pigments de colorants organiques avec de l'aluminium, du baryum, du calcium, du strontium, du zirconium, et de leurs mélanges.

23. Préparation contenant des lipides selon la revendication 21 ou 22, **caractérisée en ce que** les fractions en quantité des pigments sont comprises dans une plage allant de 0,1 à 25 % en poids, de préférence dans une plage comprise entre 5 et 20 % en poids, et de façon tout particulièrement préférée dans une plage comprise entre 8 et 15 % en poids.

24. Préparation contenant des lipides selon l'une des revendications précédentes, **caractérisée en ce qu'**elle contient, dans une utilisation en tant qu'agent de protection solaire, du dioxyde de titane et/ou de l'oxyde de zinc sous la forme connu sous la désignation de nanopigment, qui présentent des tailles de particules dans la plage de 5 à 25 nm.

25. Préparation contenant des lipides selon la revendication 24, **caractérisée en ce qu'**elle contient lesdits nanopigments en combinaison avec des substances qui filtrent la lumière UVA ou UVB oléosolubles usuelles, autorisées par la législation nationale ou régionale respective.

26. Préparation contenant des lipides selon la revendication 25, **caractérisée en ce qu'**elle contient, en combinaison avec lesdits nanopigments en tant que substances qui filtrent la lumière UVA ou UVB, de préférence du 4-méthylbenzylidène-carnphre et du p-méthoxycinnamate d'isoamyle.

27. Produit qui présente une préparation contenant des lipides selon l'une des revendications précédentes, placée dans des flacons, des pots ou des tubes refermables.

28. Produit qui présente une préparation contenant des lipides selon l'une des revendications 1 à 21, placée dans un mécanisme distributeur refermable.
